# EUROPEAN PATENT APPLICATION

(11) **EP 1 752 086 A2**
(43) Date of publication of application: **14.02.2007**
(21) Application number: 06254166.9
(22) Date of filing: 08.08.2006
(51) Int. Cl.: A61B 5/00

(54) **Kinematic fluorescence measurement band**

(30) Priority: 09.08.2005 US 200705
(71) Applicant: LifeScan, Inc., Milpitas, CA 95035-6312 (US)
(72) Inventor: Hayter, Paul G, California 94040 (US)
(74) Representative: Tunstall, Christopher Stephen

(57) **Abstract**

A kinematic fluorescence measurement band for use with a fluorescent light-emitting bead implanted within a user's body includes a band configured for secure and removable positioned about a portion of the user's body, a base plate configured for attachment to the band and an optical plate. The optical plate includes a rigid member with a light emitter and a light detector attached thereto. The light emitter of the optical plate is configured for emitting light that is absorbed by the fluorescent light-emitting bead while the light detector is configured for detecting fluorescent light emitted by the fluorescent light-emitting bead. In addition, the base plate and optical plate are configured for kinematic attachment, detachment and kinematic reattachment to one another via a cone-shaped indent, a slot-shaped indent, and a flat surface independently disposed on either of the base or optical plates in opposing relationship to first, second and third spherical components disposed on the other of the base or optical plates.

## Description

### BACKGROUND OF THE INVENTION

1. Field of the Invention

This application relates, in general, to medical devices and, in particular, to medical devices and methods that employ fluorescence analytical techniques.

2. Description of the Related Art

A variety of devices and methods for monitoring (e.g., detecting and/or measuring) analytes, such as glucose, in bodily fluids are employed by both medical personnel and laypersons. For example, the use of photometric-based and electrochemical-based devices and methods for monitoring blood glucose has become widely adopted for the treatment of diabetes.

Fluorescence analytical techniques designed for detecting and measuring analytes in bodily fluids have also been reported. For example, U.S. Patent Nos. 5,342,789, 6,040,194 and 6,232,130 describe a variety of such techniques and related *in-vivo* sensors, including those adapted for the quantifying glucose concentration in blood or other bodily fluids.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:
FIG. 1 is a simplified schematic illustration depicting interaction between a fluorescent light-emitting bead, light emitter and light detector that is relevant to various embodiments of the present invention;
FIG. 2 is a simplified schematic illustration depicting interaction between a fluorescent light-emitting bead implanted in a user's body, a light emitter, and a light detector for detecting fluorescent light that is relevant to various embodiments of the present invention;
FIG. 3A is a simplified cross-sectional view of an adhesive fluorescence measurement patch according to an exemplary embodiment of the present invention removably adhered to a user's body;
FIG. 3B is a simplified schematic depicting the operative interaction of various electrical and optical components, including a light emitter and a light detector, suitable for use in the adhesive fluorescence measurement patch of FIG. 3A and other embodiments of the present invention;
FIG. 4 is simplified perspective and partial cut-away view of the adhesive fluorescence measurement patch of FIG. 3A removably adhered to a user's body (i.e., a user's forearm);
FIG. 5 is a simplified perspective and partial cut-away illustration of a kinematic adhesive fluorescence measurement patch, according to an exemplary embodiment of the present invention;
FIG. 6 is a simplified cross-sectional view of the kinematic adhesive fluorescence measurement patch of FIG. 5 (taken along line A-A) depicting the adhesive plate and optical plate thereof in an attached position;
FIG. 7 is a simplified cross-sectional view of the kinematic adhesive fluorescence measurement patch of FIG. 5 (taken along line A-A) depicting the adhesive plate and optical plate thereof in a detached position;
FIG. 8 is a simplified perspective illustration of the kinematic adhesive fluorescence measurement patch of FIG. 5 wherein the adhesive plate thereof is removably adhered to a user's body (i.e., a user's forearm);
FIG. 9 is a simplified perspective illustration of a kinematic adhesive fluorescence measurement patch according to another embodiment of the present invention;
FIG. 10 is a simplified cross-sectional view of the kinematic adhesive fluorescence measurement patch of FIG. 9 (taken along line B-B) depicting the adhesive plate and the optical plate thereof in an attached position;
FIG. 11 is a simplified cross-sectional view of the kinematic adhesive fluorescence measurement patch of FIG. 9 (taken along line B-B) depicting the adhesive plate and the optical plate thereof in a detached position;
FIG. 12 is a flow diagram depicting stages in a process for monitoring a fluorescence light-emitting bead implanted in a user's body according to an exemplary embodiment of the present invention; and
FIG. 13 is simplified perspective exploded view of a kinematic fluorescence measurement band according to an exemplary embodiment of the present invention attached to a user's forearm.

### DETAILED DESCRIPTION OF PREFERRED EMBODIMENTS

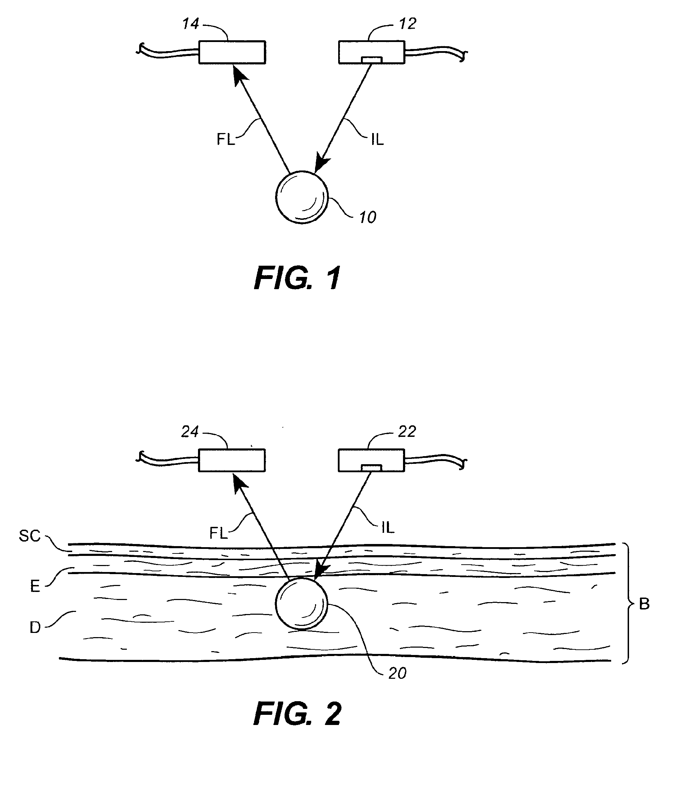

FIG. 1 is a simplified schematic illustration depicting interaction between a fluorescent light-emitting bead 10, light emitter 12 and light detector 14 that is relevant to various embodiments of the present invention. Fluorescent light-emitting bead 10 includes at least one fluorescent reactant (e.g., a fluorescent dye) that emits fluorescent light FL as a result of absorbing incident light IL (that has been emitted by light emitter 12), with characteristics of the emitted fluorescent light FL being dependent on the concentration of an analyte that is in communication with (e.g., in contact with) the fluorescent light-emitting bead. Fluorescent reactants that can be included in such a fluorescent light-emitting bead, and their behavior when in communication with an analyte, are described in U.S. Patent Nos. 5,342,789, 6,040,194, and 6,232,130, each of which is hereby fully incorporated by reference. Fluorescent light-emitting bead 10 can also include an encapsulating material such as, for example, alginate.

FIG. 2 is a simplified schematic illustration depicting interaction between a fluorescent light-emitting bead 20 implanted in a user's body B, a light emitter 22 and a light detector 24 that is relevant to various embodiments of the present invention. The portion of user's body B depicted in FIG. 2 includes a Stratum Corneum portion SC, an Epidermis portion E and Dermis portion D.

As with fluorescent light-emitting bead 10, fluorescent light-emitting bead 20 includes at least one fluorescent reactant (e.g., a fluorescent dye) that emits fluorescent light FL as a result of absorbing incident light IL (that has been emitted by light emitter 22), with characteristics of the emitted fluorescent light being dependent on the concentration of an analyte that is in communication with the fluorescent light-emitting bead.

FIG. 2 depicts fluorescent light-emitting bead 20 implanted in a user's body B. In this circumstance, incident light IL and fluorescent light FL are of a wavelength(s) and intensity such that incident light IL is able to pass through the user's body B to reach fluorescent light-emitting bead 20 and fluorescent light FL is able to pass through the user's body to reach light detector 24. Fluorescent light-emitting bead 20 includes at least one fluorescent reactant and is configured in such a way that a predetermined characteristic(s) of fluorescent light FL varies as a function of bodily fluid analyte concentration (e.g., glucose concentration) in the user' body B.

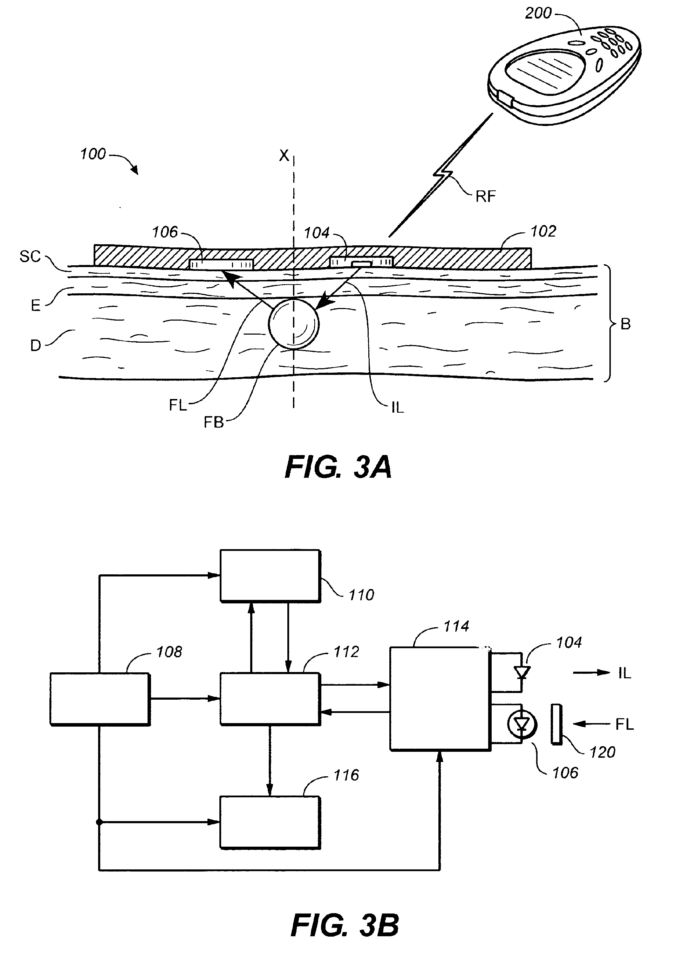

FIG. 3A is a simplified cross-sectional view of an adhesive fluorescence measurement patch 100 for use with a fluorescent light-emitting bead FB implanted within a user's body B, that includes a Stratum Corneum portion SC, an Epidermis portion E and Dermis portion D, according to an exemplary embodiment of the present invention. In FIG. 3A, adhesive fluorescence measurement patch 100 is removably adhered to a user's body B and in communication with a remote module 200 via radiofrequency signals RF. Adhesive fluorescence measurement patch 100 includes an adhesive sheet 102 configured for removable adhesion to user's body B, a light emitter 104 attached to adhesive sheet 102, and a light detector 106 also attached to adhesive sheet 102. Although FIG. 3A depicts light emitter 104 and light detector 106 embedded in adhesive sheet 102, the attachment of light emitter 104 and light detector 106 to adhesive sheet 102 can take any suitable form known to one skilled in the art.

Fluorescent light-emitting bead FB can be implanted, for example, in the range of approximately 1 mm to 4mm below the surface of a user's skin. In addition, light emitter 104 and light detector 106 can be located, for example, in the range of 0mm to 10mm above the surface of the user's skin when adhesive fluorescence measurement patch 100 is adhered to the user's body B (i.e., adhered to the user's skin).

For the sake of simplicity, FIG. 3A depicts adhesive fluorescence measurement patch 100 as including only an adhesive sheet, light emitter and light detector. However, once apprised of the present disclosure, one skilled in the art will recognize that adhesive fluorescence measurement patches, kinematic adhesive fluorescence measurement patches and kinematic adhesive fluorescence measurement bands according to the present invention can include various other components, electrical and/or optical, that provide for suitable and beneficial operation. In this regard, FIG. 3B is a simplified schematic diagram depicting the operative interaction of various electrical and optical components, including a light emitter 104 and a light detector 106, suitable for use in the adhesive fluorescence measurement patch of FIG. 3A and other embodiments of the present invention. In FIG. 3B, elements or other items common with FIG. 3A are identically labeled.

As depicted in FIG. 3B, the electrical and optical components include a power module 108, an RF transceiver module 110, a micro-controller module 112, a driver/amplifier module 114, a buzzer module 116 (for providing feedback to a user) and an optical filter module 120. Light emitter 104 can be, for example, an LED 525nm wavelength light emitter such as SMD LED part number LTST-C903TGKT available from Lite-On Corp. Light detector 106 can be, for example, light detector part number S 8745-01 available from Hamamatsu. Optical filter module 120 can include, for example, 600nm and 700nm band pass filters. Micro-controller module 112 can be, for example, an MSP 430 series micro-controller available from Texas Instruments. Power module 108 can be, for example, a rechargeable or non-rechargeable battery module. If desired, all the electrical and optical components depicted in FIG. 3B can be mounted on a printed circuit board (PCB) and the PCB attached to adhesive sheet 102.

In addition, once apprised of the present disclosure, one skilled in the art will recognize that embodiments of the present invention can be readily modified for use with suitable fluorescent light-emitting devices other than a fluorescent light-emitting bead. For example, such adhesive fluorescence measurement patches could be used with fluorescent injected oils or fluorescent tattoos as described in U.S. Patent No. 5,342,789, which is hereby fully incorporated by reference.

In FIG. 3A, fluorescent light-emitting bead FB is implanted in user's body B, and contains at least one fluorescent reactant that emits fluorescent light FL as a result of absorbing incident light IL. In addition, a characteristic(s) of fluorescent light FL varies as a function of analyte concentration in contact with fluorescent light-emitting bead FB. Therefore, adhesive fluorescence measurement patch 100, in conjunction with fluorescent light-emitting bead FB and remote module 200, can be used for measuring the concentration of an analyte (e.g., blood glucose) in the bodily fluid of a user's body.

Referring again to FIG. 3A, an imaginary optical axis X of adhesive fluorescence measurement patch 100 is depicted by a broken line. Light emitter 104 and light detector 106 are attached to adhesive sheet 102 in a predetermined relationship relative to imaginary optical axis X. In addition, imaginary optical axis X is positioned in a predetermined juxtaposition to the fluorescent light-emitting bead FB when the adhesive fluorescence measurement patch is removably adhered to a user's body (as in FIG. 3A). The predetermined juxtaposition of imaginary optical axis X and fluorescent light-emitting bead FB will typically be associated with a suitable alignment tolerance in the range of, for example, +/- 1 mm to +/- 2mm.

The predetermined relationship of light emitter 104 and light detector 106 with imaginary optical axis X and the predetermined juxtaposition of imaginary optical axis X with the fluorescent light-emitting bead FB provide for (i) emitted incident light IL from light emitter 104 to be incident on, and absorbed by, fluorescent light-emitting bead FB and (ii) fluorescent light FL emitted by fluorescent light-emitting bead FB to be detected by light detector 106 (the emitted light IL and fluorescent light FL are, for the sake of simplicity, depicted as arrows in FIG. 3A (as well as in FIGs. 1 and 2)). Therefore, adhesive fluorescence measurement patch 100 can be readily adhered to user's body B in a position that provides for incident light IL to operatively reach fluorescent light-emitting bead FB, as well as for fluorescent light FL to operatively reach light detector 106. Since light emitter 104 and light detector 106 are securely attached to adhesive sheet 102 in a proper predetermined relationship to imaginary optical axis X, an operable alignment of light emitter 104 and light detector 106 with an implanted fluorescent light-emitting bead FB is easily obtained and maintained during use.

It should be noted that although FIG. 3A depicts light emitter 104 and light detector 106 as being symmetrically disposed about imaginary optical axis X, such symmetry is not necessarily required. In addition, the predetermined relationship of light emitter 104 and light detector 106 with imaginary optical axis X, as well as the predetermined juxtaposition of imaginary optical axis X with the fluorescent light-emitting bead FB, can be such the amount of reflected light from the fluorescent light-emitting bead received by the light detector is relatively minimized while the amount of fluorescent light received by the light detector is relatively maximized.

Adhesive sheet 102 can be any suitable adhesive sheet known to those of skill in the art including, for example, adhesive sheets that include commercially available pressure sensitive adhesives. Furthermore, adhesive sheets employed in embodiments of the present invention can include a top layer and at least one adhesive lower layer disposed on at least a portion of the top layer.

The top layer and adhesive lower layer(s) employed in the adhesive sheet can be any suitable combination of single-sided adhesive layers, double-sided adhesive layers, transfer adhesive layers and non-adhesive layers. The single-sided and double-sided adhesive layers can be pressure sensitive, in that they removably adhere to a surface of a user's body when pressure is applied. Typical pressure sensitive adhesive layers include those based on acrylics, natural rubber, synthetic rubber and silicone polymers. Suitable pressure sensitive adhesive layers are commercially available from, for example, Adhesives Research, Inc., of Glen Rock, Pennsylvania under the commercial name ARcare^{®}.

The top layer and adhesive lower layer(s) of an adhesive sheet can be clear or opaque, and are typically flexible. The top layer and adhesive lower layer(s) can be made, for example, from an extruded or cast polymer film, or can be made using woven or non-woven fabric and can be elastic, or inelastic. In addition, they can be made from any suitable material, including, for example polyester, polycarbonate, polystyrene, polypropylene, polyethylene, acrylonitrile butadiene styrene (ABS), polyurethane, silicone, and woven or non-woven fabrics. Suitable polymer films and fabrics can be purchased, for example, from Tekra Corporation of New Berlin, Wisconsin.

If desired, one or more release liners can be employed to cover all or a portion of adhesive sheets employed in embodiments of the present invention. Such release liners are typically made by, for example, siliconizing polyester, polyethylene, polypropylene or paper. Release liners can also be manufactured by treating the surface of a suitable material with a fluorocarbon-based compound. Prior to use of an adhesive fluorescence measurement patch, one or all of the release liners are pealed off of the adhesive sheet. Suitable release liners are commercially available from, for example, Rexam Release, of Bedford Park, Illinois.

The adhesive sheet employed in embodiments of the present invention can be any suitable thickness. However, a typical non-limiting thickness range is from 0.0005 inches to 0.040 inches (excluding the thickness of the light emitter and light detector that are attached to the adhesive sheet). A major surface of the adhesive fluorescence measurement patch (i.e., the surface facing a user's body when the adhesive fluorescence measurement patch is adhered) can have any suitable surface area with a typical surface area being, for example, in the range of from 0.40 square inches to 4 square inches. However, larger surface areas, for example, 40 square inches, can be employed if desired.

Any suitable light emitter 104 and suitable light detector 106 known to one skilled in the art can be employed in adhesive fluorescence measurement patches according to embodiments of the present invention. Suitable light emitters can be, for example, light emitting diodes (e.g., light emitting diodes commercially available from Lite-On Technology Corporation of Milpitas, California). Suitable light detectors can be, for example, photodiodes (e.g., photodiodes commercially available from Hamamatsu Corporation of Bridgewater, New Jersey).

In FIG. 3A, adhesive fluorescence measurement patch 100 is depicted as in communication with remote module 200 via radio frequency signals RF. However, once apprised of the present disclosure, one skilled in the art will recognize that other suitable means of providing communication between an adhesive fluorescence measurement patch and a remote module can be employed, including wired communication.

Remote module 200 can have any suitable capabilities, including the capability to control of light emitter 104 and light detector 106 and the capability to process communications received from adhesive fluorescence measurement patch 100. For example, remote module 200 can have the capability to continuously or intermittently correlate fluorescent light detected by light detector 106 to analyte concentration and to then employ the correlation to control other devices, such as an insulin pump. Suitable remote controllers, as can be modified by one skilled in the art for use in embodiments of the present invention, are described in International Application No. PCT/US03/05943 (published as WO 03/071930 A2 on September 4, 2003) which is hereby fully incorporated by reference.

One skilled in the art will recognize that adhesive fluorescence measurement patch 100 is symmetrically shaped (i.e., circular in shape) in one dimension about imaginary optical axis X. However, as described below, adhesive fluorescence measurement patches according to other embodiments of the present invention can be non-symmetrically shaped (e.g., square, rectangular, oval or triangular shaped) about their imaginary optical axis.

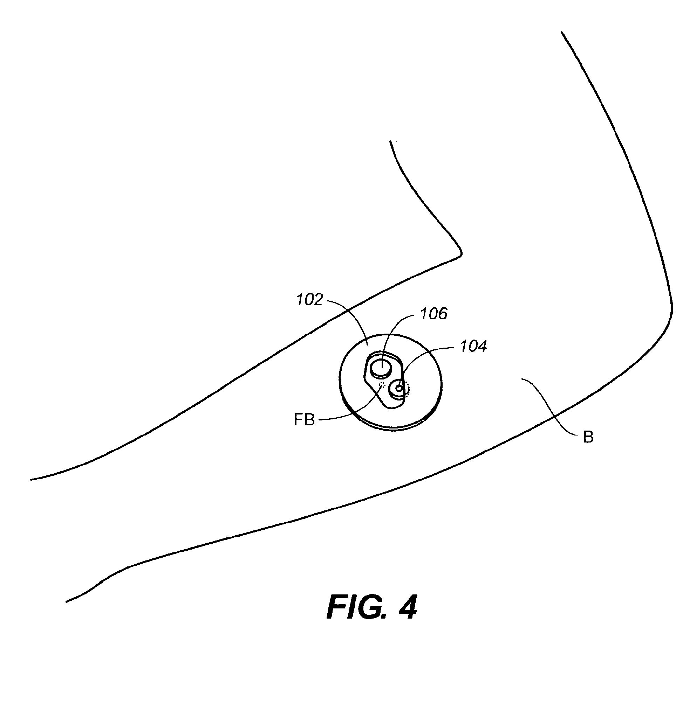

FIG. 4 is simplified perspective and partial cut-away view of the adhesive fluorescence measurement patch of FIG. 3A removably adhered to a user's body B (i.e., a user's forearm). In the embodiment of FIGs. 3A and 4, imaginary optical axis X is aligned with fluorescent light-emitting bead FB. In addition, since imaginary optical axis X passes through the center of adhesive fluorescence measurement patch 100, adhesive fluorescence measurement patch 100 is itself centered above fluorescent light-emitting bead FB when removably adhered to user's body B. However, once apprised of the present disclosure, one skilled in the art will recognize that adhesive fluorescence measurement patches according to the present invention need not necessarily be centered above a fluorescent light-emitting bead FB, as long as the positioning of the adhesive fluorescence measurement patch provides for (i) emitted incident light IL from light emitter 104 to be incident on, and absorbed by, fluorescent light-emitting bead FB and (ii) fluorescent light FL emitted by fluorescent light-emitting bead FB to be detected by light detector 106.

Since adhesive fluorescence measurement patch 100 is adhered (albeit removably) to user's body B, light emitter 104 and light detector 106 remain essentially stationary relative to fluorescent light-emitting bead FB.

When adhered to a user's body, adhesive fluorescence measurement patch 100 can be used, for example, to continuously monitor blood glucose concentration within the user's body. In this circumstance, adhesive fluorescence measurement patch 100 can be removed and replaced, as needed, during the lifetime of fluorescent light-emitting bead FB (which can range from days to months).

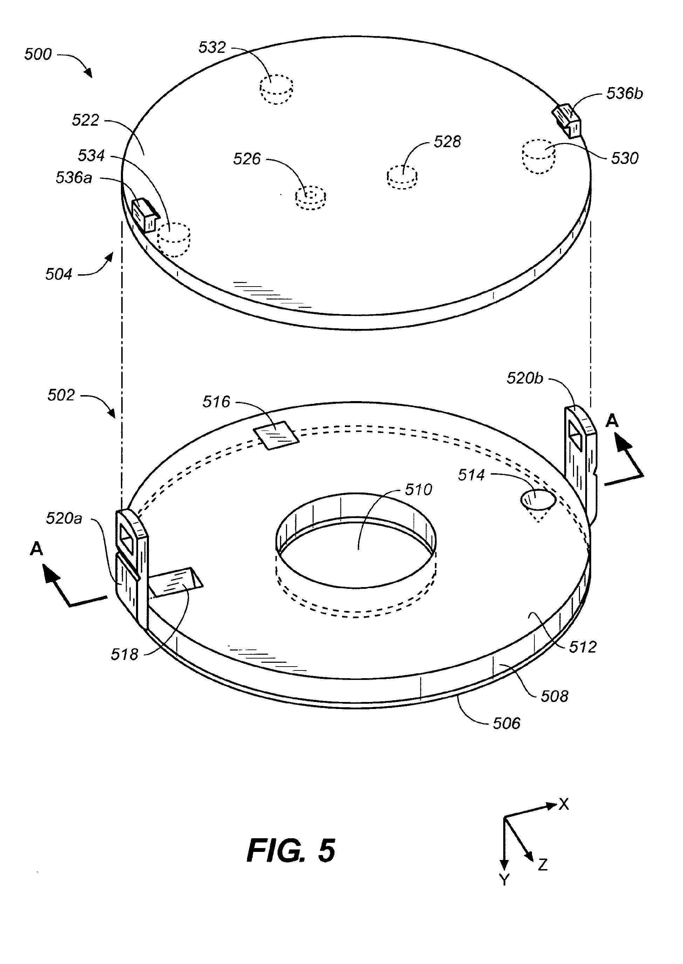

FIGs. 5, 6, 7 and 8 are various simplified depictions of a kinematic adhesive fluorescence measurement patch 500 according to an exemplary embodiment of the present invention for use with a fluorescent light-emitting bead FB implanted within a user's body (B). Kinematic adhesive fluorescence measurement patch 500 includes an adhesive plate 502 and an optical plate 504.

As is described in detail below, adhesive plate 502 and optical plate 504 are configured for rapid and precise kinematic attachment to one another, detachment from one another and kinematic reattachment to one another. It can be beneficial for a user to be able to detach and subsequently rapidly reattach the optical plate to the adhesive plate. For example, prior to bathing, a user may wish to detach and store the optical component while the adhesive plate remains adhered to the user's body, thus avoiding the need to frequently remove and subsequently realign and re-adhere the adhesive plate. After bathing, a user can rapidly and precisely reattach the optical plate to the adhesive plate in a kinematic manner, thus preserving operative alignment of the various components of the kinematic adhesive fluorescence measurement patch with an implanted fluorescent light-emitting bead. In addition, reducing the frequency at which the adhesive plate is adhered to a user's body can minimize the potential for tissue trauma.

Referring to FIGs. 5 through 8, adhesive plate 502 is configured for removable adhesion to a user's body (e.g., a user's forearm) by having included therein an adhesive layer 506 disposed on a rigid layer 508. However, once apprised of the present disclosure, one skilled in the art will recognize that adhesive plates employed in embodiments of the present invention are not limited in design to an adhesive layer disposed on a rigid layer but rather can take any suitable configuration.

Rigid layer 508 (as well as rigid member 522 described below) can be formed from any suitable material including but not limited to, for example, metal, ceramic, injection molded plastic (e.g., injection molded ABS, polycarbonate, acrylic, styrene and polyolefin) and combinations thereof. Adhesive layer 506 can be formed from any suitable material known to one skilled in the art including the pressure sensitive adhesives described above respect to the adhesive sheet of the embodiment of FIG. 3A and 4.

In the embodiment of FIGs. 5 through 8, adhesive plate 502 also includes an opening 510 therethrough, a surface 512 with cone-shaped indent 514, flat surface 516, and slot-shaped indent 518 disposed thereon, and two fastening clips 520a and 520b.

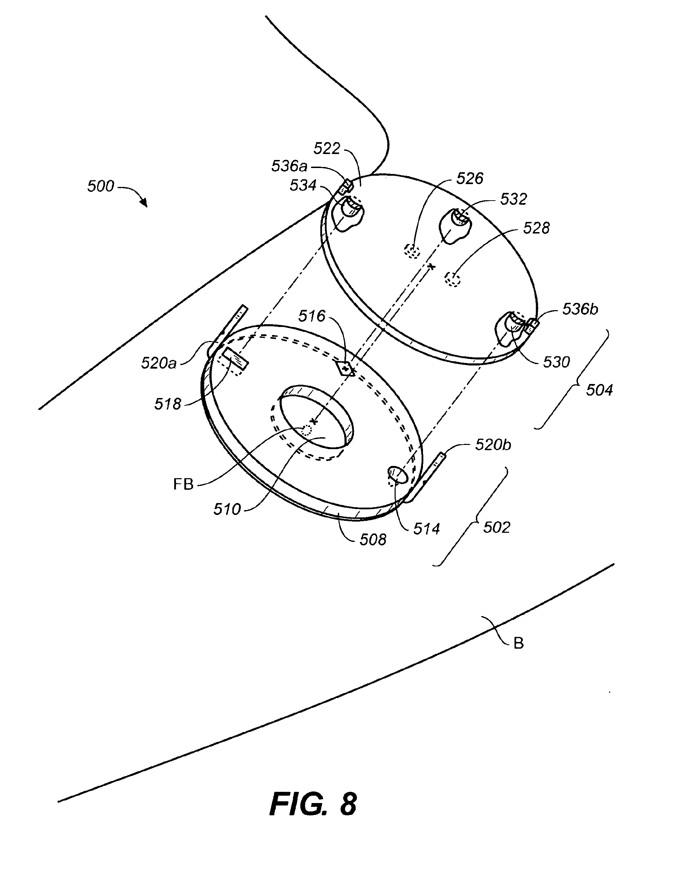

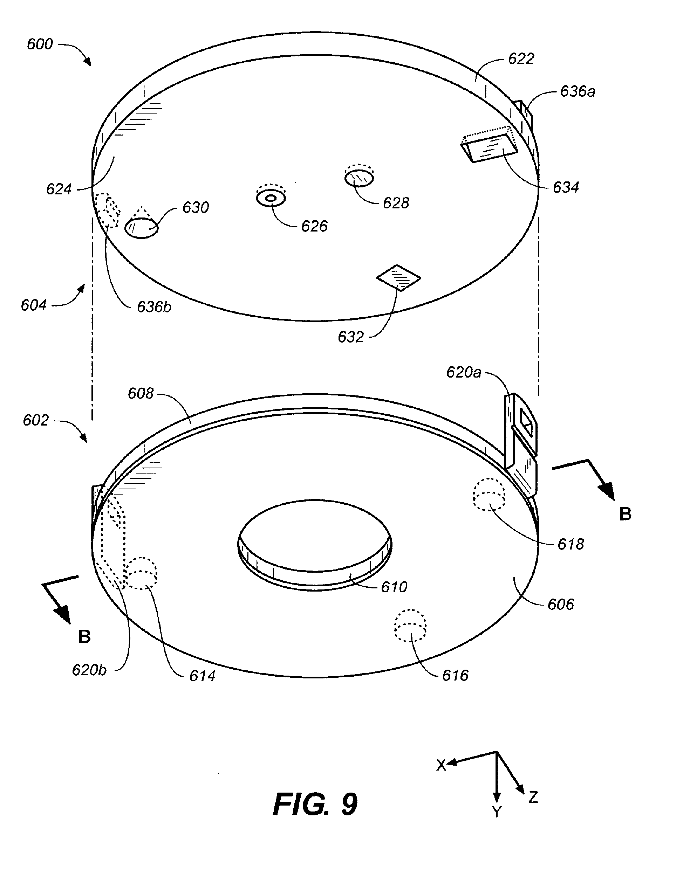

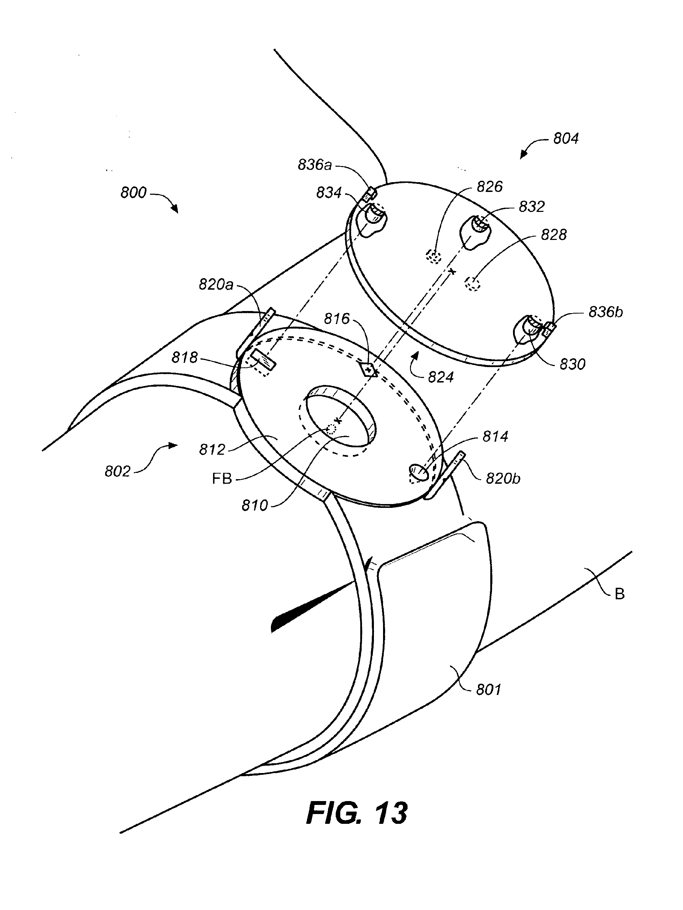

In FIGs. 5 and 8 (as well as FIGs. 9 and 13 described below), solid lines are employed to indicate a closed position of fastening clips (such as 520a and 520b) with dashed lines indicating an open position of fastening clips (such as 520a and 520b). In the closed position, fastening clips 520a and 520b provide a clamping force between adhesive plate 502 and optical plate 504. In addition, and as would be understood by one skilled in the art, dashed lines are also employed in FIGs. 5, 8, 9 and 11 to indicate features that would be hidden from view in the perspective of these FIGs. Furthermore, broken lines are employed in FIGs. 5, 7, 8, 9, 11 and 13 to indicate alignment of various elements or features of interest. For example, a dashed line terminating in a plus sign (+) at either end is employed in FIGs. 5, 8, 9 and 13 to indicate an imaginary optical axis of interest in the illustrated embodiment.

In the embodiment of FIGs. 5-9, optical plate 504 includes a rigid member 522 with a surface 524. Optical plate 504 also includes a light emitter 526 and a light detector 528, each attached to rigid member 522. Light emitter 526 is configured for emitting light that is absorbed by the fluorescent light-emitting bead FB. In this regard, the light emitter and light detector can be attached to the rigid member of the optical plate in predetermined relationship relative to an imaginary optical axis of the kinematic adhesive fluorescence measurement patch, the imaginary optical axis being positioned in a predetermined juxtaposition to the fluorescent light-emitting bead when the adhesive plate is removably adhered to a user's body and the optical plate is kinematically attached to the adhesive plate.

Optical plate 504 also includes a first hemisphere 530, second hemisphere 532, and third hemisphere 534 disposed on surface 524 and fastening posts 536a and 536b. FIG. 5 employs a partial cut-away depiction in order to clearly illustrate first hemisphere 530, second hemisphere 532 and third hemisphere 534. First hemisphere 530, second hemisphere 532 and third hemisphere 534 can be formed of any suitable material including, for example, polished and hardened steel. Once apprised of the present invention, one skilled in the art will recognize that, in general, a "spherical component" can be substituted for the depicted hemispheres including, for examples, a full sphere. In addition, although FIGs. 5-11 depict fastening clips and fastening posts, other suitable means for providing the aforementioned clamping force can be substituted for the fastening clips and posts.

In the embodiment of FIGs. 5 through 8, adhesive plate 502 and the optical plate 504 are configured for kinematic attachment to one another, detachment from one another and kinematic reattachment to one another via kinematic interaction between (i) cone-shaped indent 514, slot-shaped indent 518, and flat surface 516 disposed on surface 512 and (ii) first hemisphere 530, second hemisphere 532 and third hemisphere 534 disposed surface 524 of optical plate 504. In this regard, it should be noted that surface 524 of optical plate 504 is an opposing surface with respect to surface 512 of adhesive plate 502.

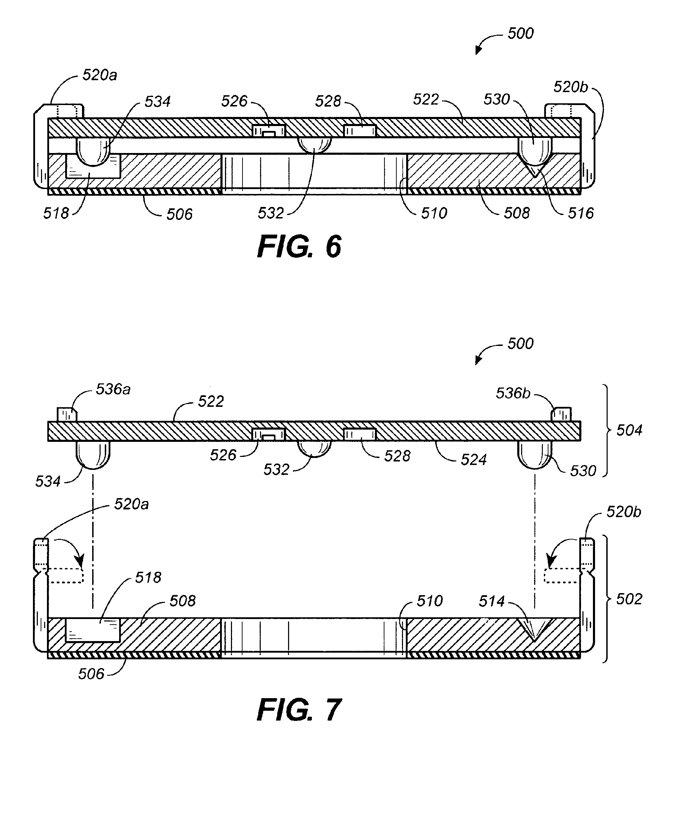

The kinematic attachment of optical plate 504 to adhesive plate 502 is accomplished as follows. Referring in particular to FIG. 6, when optical plate 504 is clamped to adhesive plate 502 by operative engagement of fastening clips 520a and 520b with fastening posts 536a and 536b, first hemisphere 530, second hemisphere 532, and third hemisphere 534 make contact with cone-shaped indent 514, flat surface 516 and slot-shaped indent 518, respectively.

Cone-shaped indent 514 provides three points of contact with first hemisphere 530, flat surface 516 provides a single point of contact with second hemisphere 532 and slot-shaped indent 518 provides two points of contact with third hemisphere 532. Therefore and thereby, cone-shaped indent 514 serves to constrain motion of optical plate 504 in the x-axis, y-axis and z-axis of the kinematic adhesive fluorescence measurement patch, while slot-shaped indent 518 serves to constrain motion around a y-axis (referred to as pitch) and a z-axis (referred to as yaw) and flat surface 532 constrains motion around the x-axis (referred to as roll). Since all six axes are constrained but only once, the attachment (and reattachment) is referred to as a kinematic attachment (and kinematic reattachment). A further description of kinematic attachment, albeit in regard to optical mounts for optical benches (typically a large rigid block supported shock absorbers), is in U.S. Patent No. 6,266,196, which is hereby fully incorporated by reference.

It should be noted that when adhesive plate 502 is removably adhered to a user's body (e.g., a user's forearm as illustrated in FIG. 7), care is taken to align the depicted imaginary optical axis in a predetermined relationship with the implanted fluorescent light-emitting bead. Thereafter, when optical plate 504 is kinematically attached or reattached to adhesive plate 502, optical plate 504 is precisely and quickly aligned for operative use (i.e., aligned in a position wherein the kinematic adhesive fluorescence measurement patch provides for (i) emitted incident light IL from light emitter 526 to be incident on, and absorbed by, fluorescent light-emitting bead FB and (ii) fluorescent light FL emitted by fluorescent light-emitting bead FB to be detected by light detector 528). During such use, emitted incident light IL and fluorescent light FL pass through opening 510. However, it should be noted that an opening (such as opening 510) is optional in that other suitable means for the incident light IL to reach fluorescent light-emitting bead FB and fluorescent light FL to be detected by light detector 528 can be provided. For example, adhesive plate 502 could be constructed of a light transparent material or be of a thickness that provides for light passage therethrough.

FIGs. 9, 10 and 11 are various simplified depictions of a kinematic adhesive fluorescence measurement patch 600 according to another embodiment of the present invention for use with a fluorescent light-emitting bead FB implanted within a user's body (B). Kinematic adhesive fluorescence measurement patch 600 includes an adhesive plate 602 and an optical plate 604.

As is described in detail below, adhesive plate 602 and optical plate 604 are configured for rapid and precise kinematic attachment to one another, detachment from one another and kinematic reattachment to one another.

Referring to FIGs. 9 through 11, adhesive plate 602 is configured for removable adhesion to a user's body (e.g., a user's forearm) by having included therein an adhesive layer 606 disposed on a rigid layer 608. In the embodiment of FIGs. 9 through 11, adhesive plate 602 also includes an opening 610 therethrough, a surface 612 with first hemisphere 614, second hemisphere 616 and third hemisphere 618 disposed thereon, and two fastening clips 620a and 620b. Fastening clips 620a and 620b can be formed, for example, from plastic, spring steel or elastic bands.

In FIG. 9, solid lines are employed to indicate a closed position of fastening clips 620a and 620b with dashed lines indicating an open position of fastening clips 620a and 620b. In the closed position, fastening clips 620a and 620b provide a clamping force between adhesive plate 602 and optical plate 604.

Optical plate 604 includes a rigid member 622 with a surface 624. Optical plate 604 also includes a light emitter 626 and a light detector 628, each attached to rigid member 622. Light emitter 626 is configured for emitting light that is absorbed by the fluorescent light-emitting bead FB and light detector 628 is configured for detecting fluorescent light emitted by fluorescent light-emitting bead FB.

Optical plate 604 also includes a cone-shaped indent 630, flat surface 632, and slot-shaped indent 634 disposed on surface 624, and fastening posts 636a and 636b.

Adhesive plate 602 and the optical plate 604 are configured for kinematic attachment to one another, detachment from one another and kinematic reattachment to one another via kinematic interaction between (i) cone-shaped indent 630, slot-shaped indent 634, and flat surface 632 disposed on surface 624 of optical plate 604 and (ii) first hemisphere 630, second hemisphere 632 and third hemisphere 634 disposed surface 612 of optical plate 603. In this regard, it should be noted that surface 612 of adhesive plate 602 is an opposing surface with respect to surface 624 of optical plate 604.

It is evident from a comparison of kinematic adhesive fluorescence measurement patches 500 and 600 that they differ in the placement of (a) the cone-shaped indent, slot-shaped indent and flat surface and (b) the first, second and third hemispheres. In kinematic adhesive fluorescence measurement patch 500, the cone-shaped indent, slot-shaped indent and flat surface are included in the adhesive plate and the first, second and third hemispheres are included in the optical plate. In contrast, in kinematic adhesive fluorescence measurement patch 600, the cone-shaped indent, slot-shaped indent and flat surface are included in the optical plate and the first, second and third hemispheres are included in the adhesive plate.

This comparison illustrates that in general, the adhesive and optical plates of kinematic adhesive fluorescence measurement patches according to embodiments of the present invention are configured for kinematic attachment to one another, detachment from one another and kinematic reattachment to one another via a cone-shaped indent, a slot-shaped indent and a flat surface independently disposed on a surface of either of the adhesive plate and the optical plate (i.e., a first surface of either the adhesive plate or the optical plate) in an opposing relationship to a first spherical component, a second spherical component and a third spherical component, respectively, disposed on an opposing surface of the other of the adhesive and optical plates. In other words, each of the cone-shaped indent, slot-shaped indent and flat surface are disposed in an opposing relationship to a spherical component, but the cone-shaped indent, a slot-shaped indent and a flat surface need not necessarily all be on the same surface. Therefore, there are eight possible permutations for disposition of the cone-shaped indent, a slot-shaped indent, flat surface and first, second and third spherical components on the adhesive and optical plates

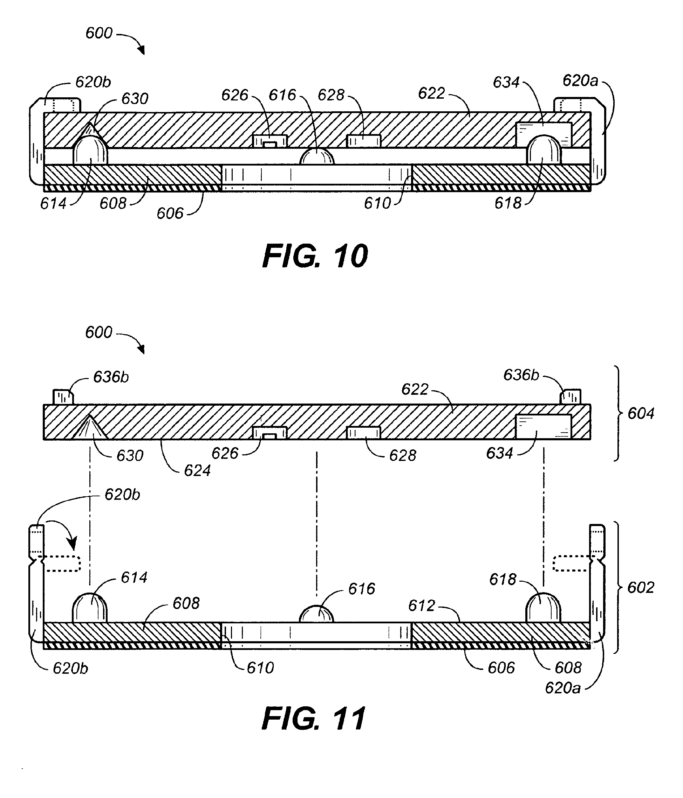

The kinematic attachment of optical plate 604 to adhesive plate 602 is accomplished as follows. Referring in particular to FIG. 11, when optical plate 604 is clamped to adhesive plate 602 by operative engagement of fastening clips 620a and 620b with fastening posts 636a and 636b, first hemisphere 614, second hemisphere 616, and third hemisphere 618 make contact with cone-shaped indent 630, flat surface 632 and slot-shaped indent 634, respectively, in a kinematic manner. Such kinematic contact and its benefits were described above with respect to the embodiment of FIGs. 5-8.

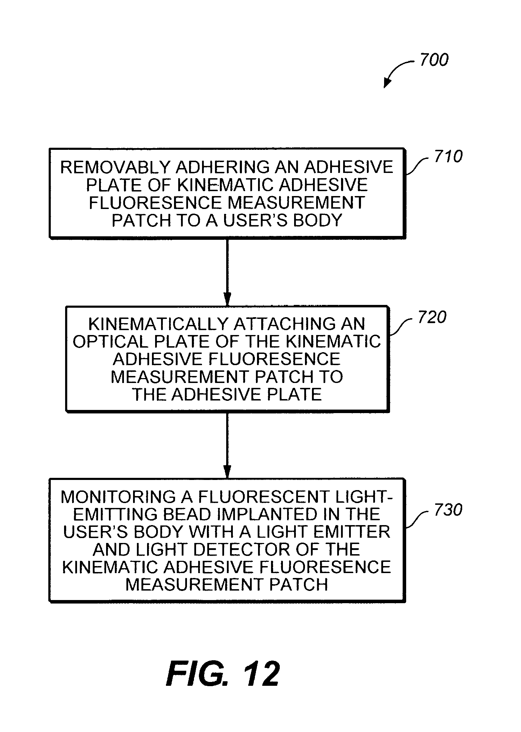

FIG. 12 is a flow diagram depicting stages in a method 700 for monitoring a fluorescent light-emitting bead implanted in a user's body according to an exemplary embodiment of the present invention. Method 700 includes removably adhering an adhesive plate of a kinematic adhesive fluorescence measurement patch to the user's body, as set forth in step 710. The kinematic adhesive fluorescence measurement patch employed in step 710 can be any suitable adhesive fluorescence measurement patch described herein.

Subsequently, an optical plate of the kinematic adhesive fluorescence measurement patch is kinematically attached to the adhesive plate such that the kinematic adhesive fluorescence measurement patch (i.e., the adhesive plate and attached optical plate) is in operative alignment with the fluorescent light-emitting bead, as set forth in step 720.

Thereafter, at step 730, the fluorescent light-emitting bead implanted in the user's body is monitored by emitting incident light from a light emitter of the kinematic adhesive fluorescent measurement patch and detecting fluorescent light emitted from the fluorescent light-emitting bead with a light detector of the kinematic adhesive fluorescent measurement patch. If desired, method 700 can also include detaching the optical plate from the adhesive plate and subsequently reattaching the optical plate to the adhesive plate in a kinematic manner.

FIG. 13 is an illustration of a kinematic fluorescence measurement band 800 for use with a fluorescent light-emitting bead FB implanted within a user's body B according to an exemplary embodiment of the present invention. FIG. 13 depicts kinematic fluorescence measurement band 800 securely and removably positioned about a portion of a user's body B (namely, a user's forearm). Such positioning can be achieved, for example, by forming fluorescence measurement band 800 at least partially of (i) self fastening materials, such as Velcro^{®} brand hook and loop fasteners (sold by Velcro USA Inc. of Manchester, New Hampshire, and Coban^{™} Self-Adherent Wrap, sold by 3M Company of St. Paul, Minnesota) or (ii) of an elastic material. In addition, conventional fasteners can be employed to securely and removably position fluorescence measurement bands according to the present invention about a portion of a user's body.

Kinematic fluorescence measurement band 800 includes a band 801 configured for secure and removable positioned about a portion of the user's body, a base plate 802 configured for attachment to band 801 and an optical plate 804. Base plate 802 can be attached to band 801 in any suitable manner including by the use of fasteners, or adhesives.

Base plate 802 and optical plate 804 are configured for rapid and precise kinematic attachment to one another, detachment from one another and kinematic reattachment to one another. Base plate 802 includes an opening 810 therethrough, a surface 812 with cone-shaped indent 814, flat surface 816, and slot-shaped indent 818 disposed thereon, and two fastening clips 820a and 820b.

Optical plate 804 includes a rigid member 822 with a surface 824. Optical plate 804 also includes a light emitter 826 and a light detector 828, each attached to rigid member 822. Light emitter 826 is configured for emitting light that is absorbed by the fluorescent light-emitting bead FB and light detector 828 is configured for detecting fluorescent light emitted by fluorescent light-emitting bead FB. Optical plate 804 also includes a first hemisphere 830, second hemisphere 832 and third hemisphere 834 disposed on surface 824 and fastening posts 836a and 836b.

In the embodiment of FIG. 13, base plate 802 and the optical plate 804 are configured for kinematic attachment to one another, detachment from one another and kinematic reattachment to one another via kinematic interaction between (i) cone-shaped indent 814, slot-shaped indent 818, and flat surface 816 disposed on surface 812 and (ii) first hemisphere 830, second hemisphere 832 and third hemisphere 834 disposed surface 824 of optical plate 804. In this regard, it should be noted that surface 824 of optical plate 804 is an opposing surface with respect to surface 812 of base plate 802.

The kinematic interaction (i.e., kinematic attachment and kinematic reattachment) of base plate 802 and optical plate 804 is essentially identical to that described above with respect to kinematic adhesive fluorescence measurement patches 500 and 600. In this regard, it is noted that the cone-shaped indent, slot-shaped indent, and flat surface can be disposed on a surface of either the base plate or the optical plate with the first, second and third hemispheres being disposed on an opposing surface of the other of the base plate and optical plate. In other words, the location of the cone-shaped indent, slot-shaped indent and flat surface can be interchanged with the location of the first, second and third hemispheres.

Kinematic fluorescence measurement bands according to the present invention are beneficial in that they can be easily removed and replaced from a user's body (e.g., a user's forearm) with minimal risk of adhesive tissue trauma.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A kinematic fluorescence measurement band for use with a fluorescent light-emitting bead implanted within a user's body, the kinematic adhesive fluorescence measurement band comprising:
a band configured for secure and removable positioned about a portion of the user's body
a base plate configured for attachment to the band; and
an optical plate including:
a rigid member;
a light emitter attached to the rigid member, the light emitter configured for emitting light that is absorbed by the fluorescent light-emitting bead; and
a light detector attached to the member, the light detector configured for detecting fluorescent light emitted by the fluorescent light-emitting bead;
wherein the base plate and the optical plate are configured for kinematic attachment to one another, detachment from one another and kinematic reattachment to one another via a cone-shaped indent, a slot-shaped indent, and a flat surface independently disposed on a first surface of one of the adhesive plate and the optical plate in opposing relationship to a first spherical component, a second spherical component and a third spherical component, respectively, disposed on an opposing surface of another of the adhesive plate and the optical plate.

2. The kinematic fluorescence measurement band of claim 1. wherein the cone-shaped indent, slot-shaped indent and flat surface are disposed on a surface of the base plate and the first sphere, second sphere, and third sphere are disposed on an opposing surface of the optical plate.

3. The kinematic fluorescence measurement band of claim 1, wherein the cone-shaped indent, slot-shaped indent and flat surface are disposed on a surface of the optical plate and the first sphere, second sphere, and third sphere are disposed on an opposing surface of the base plate.

4. The kinematic fluorescence measurement band of claim 1, wherein the optical plate further includes a power module, a micro-processor module, a driver/amplifier module and a transceiver module.

5. The kinematic fluorescence measurement band of claim 1, wherein the band is formed at least partially of a self-fastening material.

6. The kinematic fluorescence measurement band of claim 1, wherein the first, second and third spherical components are hemispheres.
